# EUROPÄISCHE PATENTSCHRIFT

(11) **EP 0 991 373 B1**
(45) Veröffentlichungstag und Bekanntmachung des Hinweises auf die Patenterteilung: **15.09.2004**
(21) Anmeldenummer: 98933619.3
(22) Anmeldetag: 16.06.1998
(51) Int. Cl.: A61F 2/00

(54) **BEUTEL ZUR WENIGSTENS TEILWEISEN UMFASSUNG EINES HERZENS**
BAG FOR AT LEAST PARTIALLY ENVELOPING A HEART
SACHET DESTINE A ENVELOPPER AU MOINS PARTIELLEMENT UN COEUR

(30) Priorität: 21.06.1997 DE 19726389
(43) Veröffentlichungstag der Anmeldung: 12.04.2000
(73) Patentinhaber: Acorn Cardiovascular, Inc., St- Paul, MN 55112 (US)
(72) Erfinder: Dr. Haindl, Hans, 30974 Wennigsen (DE)
(74) Vertreter: Meissner, Bolte & Partner
(86) Internationale Anmeldenummer: PCT/EP1998/003619
(87) Internationale Veröffentlichungsnummer: WO 1998/058598

(56) Entgegenhaltungen:
- WO-A-96/16601
- WO-A-98/14136
- FR-A- 2 737 106
- US-A- 3 983 863
- US-A- 4 690 134
- US-A- 5 702 343

## Beschreibung

Die Erfindung betrifft einen Beutel zur wenigstens teilweisen Umfassung eines Herzens.

Entzündliche Erkrankungen des Herzmuskels sowohl durch Virusinfektionen als auch durch Autoimmunprozesse können dazu führen, daß sich das Herzvolumen vergrößert. Geschieht dies über ein kritisches Maß hinaus, so kommt es zu einer progredienten Herzdilatation, die durch das Laplace'sche Gesetz zu erklären ist. Mit der Vergrößerung des Volumens des durch die linke Herzkammer gebildeten Hohlkörpers nehmen die Spannungen in der Wand dieses Hohlkörpers zu. Dies führt zu einer Überbeanspruchung der Muskelfibrillen und zum Verlassen des idealen Dehnungsbereiches der Muskelfibrillen. Im Stadium dieser Überdehnung verbleibt in der Regel ein Restvolumen im Herzen. Die Muskelfibrillen müssen nun gegen eine primär höhere Wandspannung anarbeiten, was zu ihrer weiteren Dehnung führt. Hierdurch entsteht ein Circulus vitiosus, der zu einer zunehmenden Überdehnung des Herzens mit daraus folgender Herzinsuffizienz führt.

Es ist zwar grundsätzlich möglich, diese Entwicklung in frühen Stadien durch medikamentöse Senkung der Vorlast durch ACE-Hemmer zu behandeln, jedoch ist das nicht immer erfolgreich. Außerdem wird häufig wegen zunächst geringer klinischer Auswirkungen der Zustand erst dann bemerkt, wenn ein kritisches Maß bereits überschritten ist. Als mögliche Behandlung steht dann oft nur noch die Herztransplantation zur Verfügung.

Durch DE 295 17 393 U1 ist ein Beutel der im Oberbegriff des Anspruchs 1 genannten Art bekannt, der nicht dehnbar ist und durch den eine Dilatation des Myokards durch den enddiastolischen Druck vermieden werden soll. Dieser bekannte Beutel vermeidet zwar eine Überdehnung der Wandung des Herzens, jedoch entfaltet er diese Wirkung schlagartig, wenn das Volumen des Herzens das von dem Beutel umschlossene Volumen erreicht. Diese schlagartige Wirkung wirkt sich nachteilig auf das Herz aus. Außerdem kann es zur Bildung von Falten in dem Beutel kommen, wenn das Volumen des Herzens kleiner ist als das Volumen, das durch die Abmessung des Beutels vorgegegen ist.

Der Erfindung liegt die Aufgabe zugrunde, einen Beutel zur wenigstens teilweisen Umfassung eines Herzens zu schaffen, der einer Überdehnung des Herzens entgegenwirkt, ohne daß dies in seiner Funktion beinträchtigt wird.

Die der Erfindung zugrundeliegende Aufgabe wird durch die im Kennzeichen des Anspruchs 1 angegebene Lehre gelöst.

Der Grundgedanke der Erfindung besteht darin, die Spannungen in der Wandung des vor allem durch die linke Herzkammer gebildeten Hohlkörpers teilweise aufzunehmen und so die Muskelfibrillen zu entlasten. Diese allgemeine Wirkung allein kann bereits dazu führen, daß eine Vergrößerung des Herzvolumens über ein kritisches Maß vermieden wird. Der erfindungsgemäße Beutel hat somit eine unterstützende Wirkung.

Die erfindungsgemäße Nachgiebigkeit des Beutels kann in verschiedener Weise ausgebildet sein. Eine einfachste Form besteht darin, daß der Beutel im wesentlichen unabhängig von seiner Dehnung immer die gleiche Kraft auf das Herz ausübt, so daß dieses unabhängig von seinem Volumen immer mit im wesentlichen gleicher Spannung entlastet ist. Eine andere zweckmäßige Ausführungsform der Erfindung besteht darin, daß die Wandung des Beutels elastisch ist, so daß die von ihm ausgeübte Spannung und damit Entlastung des Herzens mit zunehmendem Volumen größer wird. Die Dehnungscharakteristik kann dabei einen je nach der gewünschten Entlastung unterschiedlichen Verlauf haben. Zweckmäßig ist es z.B., daß die Elastizität der Wandung des Beutels mit zunehmender Dehnung abnimmt, um so der spezifischen Spannung in der Wandung des Herzens Rechnung zu tragen. Auch ist es vorteilhaft, wenn die Dehnung einen Grenzwert hat, bei dessen Erreichen eine weitere Vergrößerung des Herzens nicht mehr erfolgen kann. Im Gegensatz zu dem bekannten Beutel wird dieser Grenzwert aufgrund der erfindungsgemäßen Nachgiebigkeit des Beutels nicht schlagartig, sondern allmählich erreicht, so daß stoßartige Wirkungen des Beutels ausgeschlossen sind. Der Grenzwert des Beutels sollte zweckmäßigerweise bei einem Volumen des Beutels liegen, der dem Volumen des Herzens in der Phase maximaler diastolischer Füllung entspricht. Insgesamt gesehen kann also durch die Art der Nachgiebigkeit und des Verlaufs der Dehnungskurve des Beutels die durch den Beutel bewirkte Entlastung der Muskelfibrillen bestimmt und angepaßt werden.

Zur Anbringung des erfindungsgemäßen Beutels kann auf thorakoskopischem Wege der Herzbeutel eröffnet und dann der erfindungsgemäße Beutel über den Herzmuskel gezogen werden. Dies erfolgt zweckmäßigerweise etwa bis zum Anulus fibrosus, also der Klappenebene, wo der Beutel fixiert wird.

Gemäß einer Weiterbildung der Erfindung ist das Volumen des Beutels im ungedehnten Zustand kleiner als das Volumen des Herzens in der Phase minimaler Füllung. Dadurch ist sichergestellt, daß der Beutel in allen Dehnungsphasen am Herzen anliegt.

Ist gemäß einer Ausführungsform der Erfindung die Wandung des Beutels elastisch und hat dabei die Dehnung einen Grenzwert, so ist es vorteilhaft, wenn der Beutel elastisches und unelastisches Material aufweist. Das elastische Material kann dabei den Dehnungsverlauf bestimmen, während das unelastische Material den Grenzwert der Dehnung bestimmen kann. Zur praktischen Durchführung dieser Ausführungsform ist es zweckmäßig, wenn das elastische Material aus Folie oder einem Gewebe oder Gewirke aus Fäden besteht, in die bzw. in das Fäden aus im wesentlichen unelastischem Material eingelagert sind. Die Fäden aus im wesentlichen unelastischem Material sind zweckmäßigerweise längsbeweglich in der Folie bzw. dem Gewebe oder Gewirke eingelagert.

Besonders vorteilhaft ist es bei dieser Ausführungsform, daß die Fäden aus im wesentlichen unelastischem Material abschnittweise aus dem Beutel herausgeführt und so in der Länge durch abschnittweises Verknoten einstellbar und Volumen und/oder Form des Beutels dem Volumen und/oder der Form des Beutels bei maximaler diastolischer Füllung angepaßt werden. Die Fäden aus im wesentlichen unelastischem Material erstrekken sich dabei zweckmäßigerweise von dem Rand der Öffnung des Beutels zu einer im wesentlichen gegenüberliegenden Spitze des Beutels. Im Bereich der Spitze des Beutels können die Fäden dann aus diesem herausgeführt sein.

Bei der Ausführungsform, bei der die elastische Dehnung des Beutels einen Grenzwert hat, ist es zweckmäßig, wenn der Beutel aus einem Gewebe oder Gewirke aus Fäden besteht, die nicht dehnbar, jedoch biegsam und quer zu ihrer Längsausdehnung gekräuselt, insbesondere wellenförmig und/oder zickzackförmig verformt sind. Durch die Art der Verformung läßt sich der Dehnungsverlauf und der Grenzwert bestimmen.

Die Wandung des Beutels besteht zweckmäßigerweise aus vorzugsweise thermoplastischem Kunststoff, der eine einfache Verformung und Anpassung der Form des Beutels an die Form des Herzens ermöglicht, oder aus biologischem Material, für das sich besonders gut denaturiertes Rinderpericard eignet.

Um die Durchlässigkeit für Gas, insbesondere Sauerstoff, und für Flüssigkeit zu erreichen, ist die Wandung des erfindungsgemäßen Beutels zweckmäßigerweise als Netz ausgebildet. Dieses kann in zweckmäßiger Weise aus offenporigem Schaumstoff, beispielsweise Silikonschaumstoff, bestehen. Ein solcher Schaumstoff ist in der Lage, für eine sehr gleichmäßige und schonende Aufbringung des Druckes auf den Herzmuskel zu sorgen. Ausserdem hat ein solcher Schaumstoff die Fähigkeit, ein Gleitmittel aufzunehmen, beispielsweise seröse Flüssigkeit, so daß eine gute Gleitfähigkeit zwischen Beutel und Pericard besteht. Das Gleitmittel ist zweckmäßigerweise ein biologisches Gleitmittel, wozu sich besonders gut eine gentechnisch hergestellte Hyaluronsäure eignet. Durch die vorherige Einbringung eines Gleitmittels in den Schaumstoff ist eine gute Gleitfähigkeit von Anfang an gegeben, wodurch eine primäre, sich selbst verstärkende Reizung des Pericards vermieden ist.

Besteht die Wandung des Beutels erfindungsgemäß aus einem Netz, so kann dies in zweckmäßiger Weise durch eine mit Durchbrüchen versehene Folie gebildet sein. Eine solche Folie ist in der Lage, großflächig den Druck des Beutels auf das Herz zu übertragen.

Das die Wandung des erfindungsgemäßen Beutels bildende Netz kann auch aus einem Gewebe oder Gewirke bestehen. Auf diese Weise ist das Dehnungsverhalten des Beutels in weiten Grenzen den jeweiligen Gegebenheiten anzupassen.

Ganz gleich, ob das die Wandung des Beutels bildende Netz aus Folie, einem Gewirke oder einem Gewebe besteht, immer ist es zweckmäßig, zusätzlich eine Beschichtung mit offenporigem Schaumstoff vorzusehen, der eine gleichmäßige Übertragung der Kräfte gewährleistet und außerdem in der Lage ist, ein Gleitmittel aufzunehmen.

Nimmt die Elastizität der Wandung des Beutels mit zunehmender Dehnung ab oder hat die Dehnung einen Grenzwert, so besteht eine besonders zweckmäßige Ausführungsform der Erfindung darin, daß der Beutel aus zwei Arten von Fäden oder Fasern aus Kunststoff besteht, von denen die eine Art eine höhere, vorzugsweise wesentlich höhere Verformungstemperatur als die andere Art hat und wobei die eine Art elastisch und die andere Art demgegenüber weniger, vorzugsweise wesentlich weniger elastisch ist. Bei Verwendung derart unterschiedlicher Fasern ist es möglich, die Form des Beutels durch thermoplastische Verformung bei einer Temperatur zu erreichen, bei der das weniger oder nicht elastische Material bei einer vorgegebenen Verformungstemperatur bleibend verformt wird, nicht jedoch das elastischere Material. Das weniger oder nicht elastische Material bestimmt so die maximale Ausdehnung des Beutels, während das elastische und nicht bleibend verformte Material unterhalb der maximalen, durch das weniger oder nicht elastische Material bestimmten Form einengende Kräfte auf das Herz ausübt.

Der für die Herstellung des Beutels verwendete Kunststoff ist gemäß einer Weiterbildung der Erfindung thermoplastisch. Dies hat den Vorteil, daß der Beutel nicht nur einfach in eine vorgefertigte Form gebracht werden kann, vielmehr ist es auch möglich, den Beutel vor oder während der Operation zu formen oder seine Form zu ändern, um ihn so den vorgefundenen Dimensionen des zu umschließenden Teiles des Herzens anzupassen.

Gemäß einer Weiterbildung der Erfindung besteht der Schaumstoff aus Silikon.

Der Erfindung liegt auch die Aufgabe zugrunde, ein Verfahren zur Herstellung eines Beutels gemäß Anspruch 1 anzugeben. Die Lösung dieser Aufgabe besteht darin, daß ein Formkörper in Form des zu umfassenden Teiles des Herzens hergestellt und eine gas- und/oder flüssigkeitsdurchlässige Folie oder ein Netz oder Gewirke aus thermoplastischem Kunststoff unter Anwendung von Wärme über die Form gezogen wird.

Die Form des Formkörpers wird zweckmäßigerweise dadurch erzeugt oder bestimmt, daß die Form des zu umschließenden Herzens abgebildet und anhand dieser Abbildung der Formkörper erzeugt wird. Die Abbildung kann in beliebiger Weise, z.B. durch Röntgen- oder computertomografische Abbildung erfolgen.

Anhand der Zeichnung soll die Erfindung näher erläutert werden.
- Fig. 1: zeigt ein erstes Ausführungsbeispiel der Erfindung,
- Fig. 2: zeigt ein zweites Ausführungsbeispiel der Erfindung und
- Fig. 3: zeigt ein drittes Ausführungsbeispiel der Erfindung.

Die Zeichnung zeigt schematisch ein Herz 1, das teilweise von einem Beutel 2 umfaßt ist, dessen Wandung aus einem Netz 3 besteht. Der Beutel 2 erstreckt sich bis in den Bereich des Anulus fibrosus, also der Klappenebene, und ist dort entlang einer Abschlußkante 4 am Herzmuskel fixiert, was in der Zeichnung nicht dargestellt ist. Das Netz 3 besteht aus elastisch nachgiebigen Fäden. Das Volumen des Beutels 2 ist im ungedehnten Zustand kleiner als das Volumen des Herzens 1 in der Phase minimaler Füllung. Dadurch ist sichergestellt, daß in allen Dehnungsphasen das Netz an der Wandung des Herzens 1 anliegt.

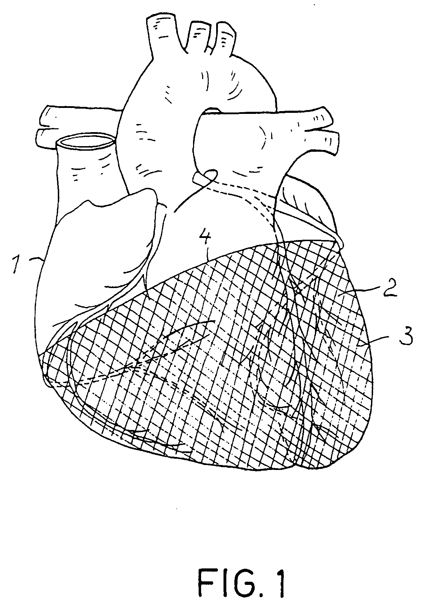

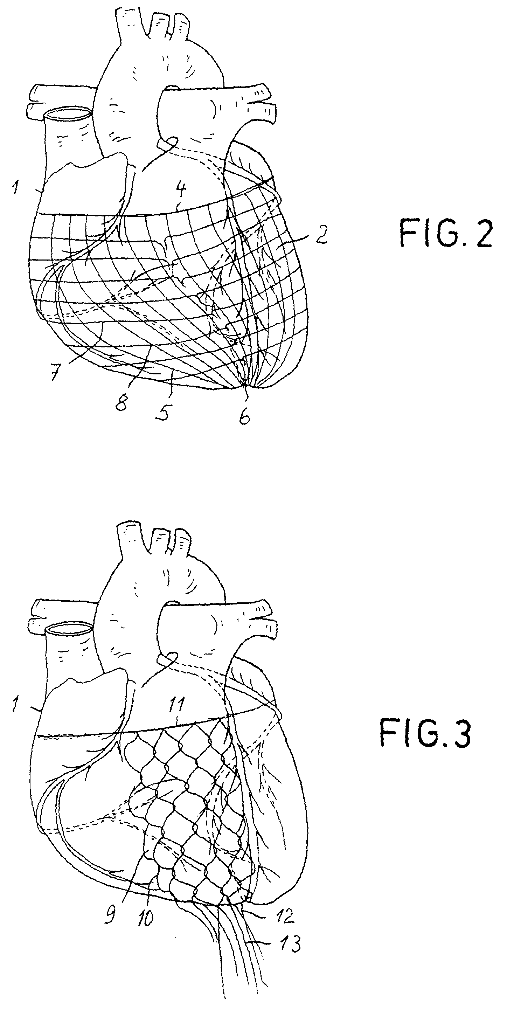

Fig. 2 zeigt eine zweite Ausführungsform der Erfindung, die eine Abwandlung der Ausführungsform gemäß Fig. 1 darstellt. Gleiche oder sich entsprechende Teile sind mit gleichen Bezugsziffern versehen. Der Unterschied besteht darin, daß Fäden 5 von der Abschlußkante 4 zu einem zentralen Punkt 6 zusammenlaufen, während Fäden 7 im wesentlichen in Umfangsrichtung verlaufen. An Kreuzungspunkten 8 sind die Fäden 5 und 7 miteinander verbunden, und zwar entweder durch Verschweißung, Verklebung oder durch Eintauchen des gesamten Beutels 2 in eine Masse, beispielsweise Schaummaterial, und nachträgliches Verfestigen desselben.

Fig. 3 zeigt ein Ausführungsbeispiel eines Beutels 9, der aus einem Gewirke von Fäden 10 besteht, die von einer Abschlußkante 11 zu einem zentralen Punkt 12 verlaufen und dort mit ihren Enden 13 herausgeführt sind, die nach Applikation des Beutels 9 in gewünschter Weise straffgezogen und miteinander verknotet werden können, um so den Beutel 9 der Form und dem Volumen des Herzens 1 anzupassen.

## Patentansprüche

1. Beutel (2) zur abstandslosen, wenigstens teilweisen Umfassung eines Herzens (1), **dadurch gekennzeichnet, daß** die Wandung des Beutels (2) elastisch ist, derart, daß die von dem Beutel auf das Herz ausgeübte Spannung mit zunehmendem Volumen des Herzens größer wird, und daß der Beutel in allen Dehnungsphasen am Herzen anliegt, derart, daß er die Spannungen in den Wandungen des vor allem durch die linke Herzkammer gebildeten Hohlkörpers teilweise aufnimmt und so die Muskelfibrillen entlastet und unterstützt.

2. Beutel nach Anspruch 1, **dadurch gekennzeichnet, daß** die Elastizität der Wandung des Beutels (2) mit zunehmender Dehnung abnimmt.

3. Beutel nach Anspruch 2, **dadurch gekennzeichnet, daß** die Dehnung einen Grenzwert hat.

4. Beutel nach Anspruch 3, **dadurch gekennzeichnet, daß** der Grenzwert bei einem Volumen des Beutels liegt, der dem Volumen des Herzens in der Phase maximaler diastolischer Füllung entspricht.

5. Beutel nach Anspruch 1, **dadurch gekennzeichnet, daß** das Volumen des Beutels im gedehnten Zustand höchstens dem Volumen des Herzens in der Phase maximaler diastolischer Füllung entspricht.

6. Beutel nach Anspruch 3, **dadurch gekennzeichnet, daß** der Beutel elastisches und unelastisches Material aufweist.

7. Beutel nach Anspruch 6, **dadurch gekennzeichnet, daß** das elastische Material aus Folie oder einem Gewebe oder Gewirke aus Fäden besteht, in die bzw. in das Fäden aus im wesentlichen unelastischem Material eingelagert sind.

8. Beutel nach Anspruch 7, **dadurch gekennzeichnet, daß** die Fäden aus im wesentlichen unelastischem Material längsbeweglich in der Folie bzw. dem Gewebe oder Gewirke eingelagert sind.

9. Beutel nach Anspruch 8, **dadurch gekennzeichnet, daß** die Fäden aus im wesentlichen unelastischem Material abschnittweise aus dem Beutel herausgeführt und so in der Länge durch abschnittweises Verknoten einstellbar und Volumen und/oder Form des Beutels dem Volumen und/oder der Form des Herzens bei maximaler diastolischer Füllung anpaßbar sind.

10. Beutel nach Anspruch 9, **dadurch gekennzeichnet, daß** sich die Fäden aus im wesentlichen unelastischem Material von dem Rand der Öffnung des Beutels zu einer im wesentlichen gegenüberliegenden Spitze des Beutels erstrecken.

11. Beutel nach Anspruch 10, **dadurch gekennzeichnet, daß** die Fäden im Bereich der Spitze des Beutels aus diesem herausgeführt sind.

12. Beutel nach Anspruch 3, **dadurch gekennzeichnet, daß** der Beutel aus einer Folie, einem Gewebe oder Gewirke besteht, das eine Faltung, Kräuselung, Plissierung oder dergleichen aufweist.

13. Beutel nach Anspruch 3, **dadurch gekennzeichnet, daß** der Beutel aus einem Gewebe oder Gewirke aus Fäden besteht, die nicht dehnbar, jedoch biegsam und quer zu ihrer Längsausdehnung gekräuselt, insbesondere wellen- und/oder zickzackförmig verformt sind.

14. Beutel nach Anspruch 13, **dadurch gekennzeichnet, daß** die Kräuselung thermisch fixiert ist.

15. Beutel nach Anspruch 3, **dadurch gekennzeichnet, daß** der Beutel aus einem Gewirke besteht und daß die Fäden des Gewirkes nicht dehnbar, jedoch biegsam sind.

16. Beutel nach Anspruch 1, **dadurch gekennzeichnet, daß** die Wandung des Beutels (2) aus Kunststoff oder biologischem Material besteht.

17. Beutel nach Anspruch 16, **dadurch gekennzeichnet, daß** das biologische Material denaturiertes Rinderpericard ist.

18. Beutel nach Anspruch 1, **dadurch gekennzeichnet, daß** die Wandung als Netz (3) ausgebildet ist.

19. Beutel nach Anspruch 18, **dadurch gekennzeichnet, daß** das Netz (3) aus offenporigem Schaumstoff besteht.

20. Beutel nach Anspruch 18, **dadurch gekennzeichnet, daß** das Netz (3) aus einer mit Durchbrüchen versehenen Folie besteht.

21. Beutel nach Anspruch 18, **dadurch gekennzeichnet, daß** das Netz (3) aus einem Gewebe oder Gewirke besteht.

22. Beutel nach Anspruch 20 oder 21, **dadurch gekennzeichnet, daß** die Folie bzw. das Gewebe oder Gewirke mit offenporigem Schaumstoff beschichtet ist.

23. Beutel nach Anspruch 2 oder 3, **dadurch gekennzeichnet, daß** der Beutel (2) aus zwei Arten von Fäden oder Fasern aus thermoplastischem Material besteht, von denen die eine Art eine höhere, vorzugsweise wesentlich höhere Verformungstemperatur als die andere Art hat und wobei die eine Art elastisch und die andere Art demgegenüber weniger, vorzugsweise wesentlich weniger elastisch ist.

24. Beutel nach Anspruch 23, **dadurch gekennzeichnet, daß** der Kunststoff thermoplastisch ist.

25. Beutel nach Anspruch 19 oder 20, **dadurch gekennzeichnet, daß** der Schaumstoff aus Silikon besteht.

26. Beutel nach Anspruch 19 oder 22, **dadurch gekennzeichnet, daß** der Schaumstoff ein Gleitmittel aufweist.

27. Beutel nach Anspruch 19, **dadurch gekennzeichnet, daß** das Gleitmittel ein biologisches Gleitmittel ist.

28. Beutel nach Anspruch 20, **dadurch gekennzeichnet, daß** das Gleitmittel gentechnisch hergestellt ist.

29. Beutel nach Anspruch 28, **dadurch gekennzeichnet, daß** das gentechnisch hergestellte Gleitmittel Hyaluronsäure ist.

30. Verfahren zur Herstellung eines Beutels gemäß Anspruch 1, **dadurch gekennzeichnet, daß** ein Formkörper in Form des zu umfassenden Teiles des Herzens hergestellt und eine gas- und/oder flüssigkeitsdurchlässige Folie oder ein Netz oder Gewirke aus thermoplastischem Kunststoff unter Anwendung von Wärme über die Form gezogen und geformt wird.

31. Verfahren nach Anspruch 30, **dadurch gekennzeichnet, daß** die Form des zu umschließenden Herzens abgebildet und anhand dieser Abbildung der Formkörper erzeugt wird.

32. Beutel nach Anspruch 1, **dadurch gekennzeichnet, daß** die Wandung des Beutels aus Polytetrafluorethylen, insbesondere aus offenporigem Schaum aus Polytetrafluorethylen besteht.

## Claims

1. A pouch (2) for the tight, at least partial enclosure of a heart (1),
**characterised in that** the wall of the pouch (2) is elastic in such a manner that the stress exerted by the pouch on the hear becomes greater as the volume of the heart increases,
and **in that** it abuts the heart in all expansion phases of the heart in such a manner that it partially absorbs the stresses in the walls of the hollow body above all formed by the left ventricle and thus relieves and assists the muscle fibrillation.

2. A pouch according to Claim 1,
**characterised in that** the elasticity of the wall of the pouch (2) decreases as the expansion increases.

3. A pouch according to Claim 2,
**characterised in that** the expansion has a limit value.

4. A pouch according to Claim 3,
**characterised in that** the limit value lies at a volume of the pouch which corresponds to the volume of the heart in the phase of maximum diastolic filling.

5. A pouch according to Claim 1,
**characterised in that** the volume of the pouch in the expanded state corresponds at most to the volume of the heart in the phase of maximum diastolic filling.

6. A pouch according to Claim 3,
**characterised in that** the pouch comprises elastic and inelastic material.

7. A pouch according to Claim 6,
**characterised in that** the elastic material consists of a sheet or a woven fabric or knit fabric of threads into which threads made from a substantially inelastic material are intercalated.

8. A pouch according to Claim 7,
**characterised in that** the threads made from substantially inelastic material are intercalated in the sheet or in the woven fabric or knit fabric.

9. A pouch according to Claim 8,
**characterised in that** the threads made from substantially inelastic material are passed in sections out of the pouch and their length can be adjusted by knotting in sections and the volume and/or shape of the pouch can be adapted to the volume and/or shape of the heart at maximum diastolic filling.

10. A pouch according to Claim 9,
**characterised in that** the threads made from substantially inelastic material extend from the edge of the opening of the pouch to a substantially opposite tip of the pouch.

11. A pouch according to Claim 10,
**characterised in that** the thread in the region of the tip of the pouch are passed out of said pouch.

12. A pouch according to Claim 3,
**characterised in that** the pouch is made from a sheet, a woven fabric or knit fabric which comprises a folding, crimping, pleating or the like.

13. A pouch according to Claim 3,
**characterised in that** the pouch is made from a woven fabric or a knit fabric from threads which are not elastic but flexible and are crimped transversely to their longitudinal extension, in particular are deformed in wavy or zig-zag fashion.

14. A pouch according to Claim 13,
**characterised in that** the crimping is thermally fixed.

15. A pouch according to Claim 3,
**characterised in that** the pouch is made from a knit fabric and **in that** the threads of the knit fabric are not elastic, but flexible.

16. A pouch according to Claim 1,
**characterised in that** the wall of the pouch (2) is made from plastic or biological material.

17. A pouch according to Claim 16,
**characterised in that** the biological material is denatured bovine pericardium.

18. A pouch according to Claim 1,
**characterised in that** the wall is constructed as netting (3).

19. A pouch according to Claim 18,
**characterised in that** the netting (3) is made from open-pore foamed material.

20. A pouch according to Claim 18,
**characterised in that** the netting (3) is made from a sheet provided with openings.

21. A pouch according to Claim 18,
**characterised in that** the netting (3) is made from a woven fabric or a knit fabric.

22. A pouch according to Claim 20 or 21,
**characterised in that** the sheet or the woven fabric or knit fabric is coated with open-pore foamed material.

23. A pouch according to Claim 2 or 3,
**characterised in that** the pouch (2) is made from two kinds of threads or fibres made from thermoplastic material, the one kind of which has a higher, preferably substantially higher, deformation temperature than the other kind, and the one kind being elastic and the other kind, on the other hand, being less, preferably substantially less, elastic.

24. A pouch according to Claim 23,
**characterised in that** the plastic is thermoplastic.

25. A pouch according to Claim 19 or 20,
**characterised in that** the foamed material is made from silicon.

26. A pouch according to Claim 19 or 22,
**characterised in that** the foamed material comprises a lubricant.

27. A pouch according to Claim 19,
**characterised in that** the lubricant is a biological lubricant.

28. A pouch according to Claim 20,
**characterised in that** the lubricant is produced by gene technology.

29. A pouch according to Claim 28,
**characterised in that** the lubricant produced by gene technology is hyaluronic acid.

30. A process for the manufacture of a pouch according to Claim 1,
**characterised in that** a shaped body in the form of the part of the heart to be enclosed is produced and a gas-permeable and/or liquid-permeable sheet or netting or a knit fabric made from thermoplastic plastic is drawn and shaped by the application of heat via the mould.

31. A process according to Claim 30,
**characterised in that** the shape of the heart to be enclosed is mapped and the shaped body is produced by means of this mapping.

32. A pouch according to Claim 1,
**characterised in that** the wall of the pouch is made from polytetrafluor ethylene, in particular from open-pore foamed material made from polytetrafluor ethylene.

## Revendications

1. Poche (2) pour entourer étroitement, au moins partiellement un coeur (1), **caractérisée en ce que** la paroi de la poche (2) est élastique de manière que la contrainte exercée par la poche sur le coeur augmente lorsqu'augmente le volume du coeur, et **en ce que** la poche s'applique contre le coeur dans toutes les phases de dilatation, de manière qu'il absorbe au moins en partie les contraintes dans les parois du corps creux formé notamment par la chambre gauche du coeur, et soulage et soutient ainsi les fibrilles musculaires.

2. Poche selon la revendication 1, **caractérisée en ce que** l'élasticité de la paroi de la poche (2) diminue lorsqu'augmente la dilatation.

3. Poche selon la revendication 2, **caractérisée en ce que** la dilatation a une valeur limite.

4. Poche selon la revendication 3, **caractérisée en ce que** la valeur limite se situe à un volume de la poche qui correspond au volume du coeur dans la phase de remplissage diastolique maximale.

5. Poche selon la revendicationl, **caractérisée en ce que** le volume de la poche à l'état dilaté correspond au maximum du volume du coeur pendant la phase de remplissage diastolique maximal.

6. Poche selon la revendication 3, **caractérisée en ce que** la poche présente une matière élastique et une matière non élastique.

7. Poche selon la revendication 6, **caractérisée en ce que** la matière élastique est constituée d'une feuille ou d'un tissu ou tricot de fil dans lequel sont intégrés des fils d'une matière sensiblement non élastique.

8. Poche selon la revendication 7, **caractérisée en ce que** les fils en matière sensiblement non élastique sont incorporés dans la feuille, le tissu ou le tricot de manière à se déplacer longitudinalement.

9. Poche selon la revendication 8, **caractérisée en ce que** les fils en matière sensiblement non élastique ressortent par endroits de la poche et sont réglables dans leur longueur par nouage par endroits, et le volume et/ou la forme de la poche peuvent être adaptés au volume et/ou à la forme du coeur lors du remplissage diastolique maximal.

10. Poche selon la revendication 9, **caractérisée en ce que** les fils en matière sensiblement non élastique s'étendent depuis le bord de l'ouverture de la poche vers une pointe sensiblement opposée de la poche.

11. Poche selon la revendication 10, **caractérisée en ce que** les fils ressortent de la poche dans la zone de sa pointe.

12. Poche selon la revendication 3, **caractérisée en ce que** la poche est constituée d'une feuille, d'un tissu ou d'un tricot qui présente un pliage, un fronçage, un plissage ou similaire.

13. Poche selon la revendication 3, **caractérisée en ce que** la poche est constituée d'un tissu ou tricot de fils qui ne sont pas extensibles mais qui sont flexibles et froncés transversalement à leur extension longitudinale, en particulier déformés de manière ondulée et/ou en zigzag.

14. Poche selon la revendication 13, **caractérisée en ce que** le fronçage est fixé thermiquement.

15. Poche selon la revendication 3, **caractérisée en ce que** la poche est constituée d'un tricot et **en ce que** les fils du tricot ne sont pas extensibles, mais flexibles.

16. Poche selon la revendication 1, **caractérisée en ce que** la paroi de la poche (2) est en matière plastique ou en matière biologique.

17. Poche selon la revendication 16, **caractérisée en ce que** la matière biologique est du péricarde de bovin dénaturé.

18. Poche selon la revendication 1, **caractérisée en ce que** la paroi est réalisée sous forme de filet (3).

19. Poche selon la revendication 18, **caractérisée en ce que** le filet (3) est constitué d'une mousse à pores ouverts.

20. Poche selon la revendication 18, **caractérisée en ce que** le filet (3) est constitué d'une feuille pourvue d'ajours.

21. Poche selon la revendication 18, **caractérisée en ce que** le filet (3) est constitué d'un tissu ou d'un tricot.

22. Poche selon la revendication 20 ou 21, **caractérisée en ce que** la feuille ou le tissu ou le tricot est revêtu d'une mousse à pores ouverts.

23. Poche selon la revendication 2 ou 3, **caractérisée en ce que** la poche 2 est constituée de deux types de fils ou fibres en matière thermoplastique dont un type présente une température de déformation supérieure, de préférence sensiblement supérieure à l'autre type, et un type étant élastique et l'autre type en revanche moins élastique, de préférence sensiblement moins élastique.

24. Poche selon la revendication 23, **caractérisée en ce que** la matière synthétique est thermoplastique.

25. Poche selon la revendication 19 ou 20, **caractérisée en ce que** la mousse est constituée de silicone.

26. Poche selon la revendication 19 ou 22, **caractérisée en ce que** la mousse présente un lubrifiant.

27. Poche selon la revendication 19, **caractérisée en ce que** le lubrifiant est un lubrifiant biologique.

28. Poche selon la revendication 20, **caractérisée en ce que** le lubrifiant est réalisé par une technique génétique.

29. Poche selon la revendication 28, **caractérisée en ce que** le lubrifiant réalisé par une technique génétique est de l'acide hyaluronique.

30. Procédé de fabrication d'une poche selon la revendication 1, **caractérisé en ce qu'**on fabrique un corps façonné sous la forme de la partie du coeur à envelopper et une feuille perméable aux gaz et/ou aux liquides ou un filet ou un tricot en matière synthétique thermoplastique est tiré sur la forme par utilisation de chaleur et formé.

31. Procédé selon la revendication 30, **caractérisé en ce que** la forme du coeur à envelopper est représentée et à l'aide de cette représentation, on produit le corps façonné.

32. Poche selon la revendication 1, **caractérisée en ce que** la paroi de la poche est constituée de polytétrafluoréthylène, en particulier d'une mousse à pores ouverts de polytétrafluoréthylène.
